(19) Europäisches Patentamt
European Patent Office
Office européen des brevets

(11) Veröffentlichungsnummer: **0 198 277**
**B1**

(12) # EUROPÄISCHE PATENTSCHRIFT

(45) Veröffentlichungstag der Patentschrift: **03.05.89**

(51) Int. Cl.⁴: **C07C 163/00**, A61K 31/165

(21) Anmeldenummer: **86104009.5**

(22) Anmeldetag: **24.03.86**

(54) **Diselenobis-benzoesäureamide primärer und sekundärer Amine, Verfahren zu ihrer Herstellung und diese enthaltende pharmazeutische Präparate.**

(30) Priorität: **12.04.85 DE 3513070**
**12.04.85 DE 3513071**

(43) Veröffentlichungstag der Anmeldung:
**22.10.86 Patentblatt 86/43**

(45) Bekanntmachung des Hinweises auf die Patenterteilung:
**03.05.89 Patentblatt 89/18**

(84) Benannte Vertragsstaaten:
**AT BE CH DE FR GB IT LI LU NL SE**

(56) Entgegenhaltungen:
**EP-A- 0 165 534**
**DE-A- 3 027 075**
**DE-C- 633 344**
**GB-A- 1 352 196**

**SOVIET INVENTIONS ILLUSTRATED, Sektion Ch, Woche E 43, 8.Dezember 1982 Derwent Publications LTD. London E 14**

(73) Patentinhaber: **A. Nattermann & Cie. GmbH, Nattermannallee 1, D-5000 Köln 30(DE)**

(72) Erfinder: **Welter, André, Dr., Grand' Route 84, B-4610 Beyne-Hevsay(BE)**
Erfinder: **Fischer, Hartmut, Dr., Arnulfstrasse 3-5, D-5000 Köln 41(DE)**
Erfinder: **Christiaens, Léon, Dr., Rue Croix André 5, B-4150 Nandrin(BE)**
Erfinder: **Wendel, Albrecht, Prof. Dr., Im Buckenloh 19, D-7400 Tübingen(DE)**
Erfinder: **Etschenberg, Eugen, Dr., Hirseweg 10, D-5000 Köln 41(DE)**
Erfinder: **Dereu, Norbert, Dr., An der Holzhecke 11, D-5020 Frechen-Bachem(DE)**
Erfinder: **Kuhl, Peter, Dr., Botzdorfer Weg 21, D-5303 Bornheim(DE)**
Erfinder: **Graf, Erich, Dr., Am Langen Hau 25, D-5014 Kerpen-Horrem(DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Diselenobis-benzoesäureamide primärer und sekundärer Amine, die sich durch wertvolle pharmakologische Eigenschaften auszeichnen, sowie Verfahren zu ihrer Herstellung und ihre Verwendung als Wirkstoff in Arzneimitteln. Sie können besonders zur Behandlung von Krankheiten Verwendung finden, die durch eine Zellschädigung aufgrund vermehrter Bildung von aktiven Sauerstoffmetaboliten hervorgerufen werden, wie z.B. Leberschäden, Herzinfarkt, Entzündungen, Psoriasis, Strahlenschäden.

Die erfindungsgemäßen Verbindungen entsprechen der allgemeinen Formel I

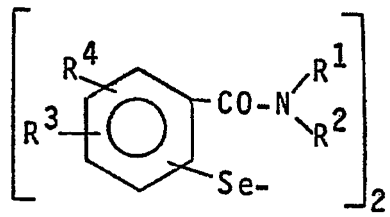

I

worin $R^1$, $R^2$ gleich oder verschieden sind mit der Maßgabe, daß mindestens ein Rest ungleich Wasserstoff sein muß, unabhängig voneinander Wasserstoff, $C_{1-18}$-Alkyl, gerade oder verzweigt, $C_{3-8}$-Cycloalkyl, $C_{3-8}$-Cycloalkylmethyl, Phenylalkyl, Phenyl, ein- bis dreimal unabhängig voneinander durch Halogen, $C_{1-4}$-Alkyl, $C_{1-4}$-Alkoxy, Hydroxy, Trifluormethyl, Nitro, Di-($C_{1-4}$-alkyl)-amino, Cyan, Carboxy, Alkoxycarbonyl, Alkoxycarbonylalkyl bzw. Methylendioxy substituiertes Phenyl bedeuten oder zusammen eine $C_4$–$C_6$-Alkylenkette bilden und $R^3$, $R^4$ gleich oder verschieden sind und unabhängig voneinander für Wasserstoff, Halogen, $C_{1-4}$-Alkyl, $C_{1-4}$-Alkoxy, Trifluormethyl, Nitro oder zusammen für Methylendioxy stehen, und die Diselenobrücke in ortho-, meta- oder para-Stellung zur Carbamidgruppe, bevorzugt in beiden Ringen steht.

Halogen bedeutet Fluor, Chlor, Brom. Als Alkylreste mit 1–4 Kohlenstoffatomen seien Methyl, Ethyl, Propyl, Isopropyl, Butyl, Isobutyl, tert-Butyl genannt, als Alkoxyreste mit 1–4 Kohlenstoffatomen kommen Methoxy, Ethoxy, Propoxy und Butoxy in Betracht.

Bevorzugt sind dabei Verbindungen, worin $R^1$, $R^2$ gleich oder verschieden, aber immer ungleich Wasserstoff sind und unabhängig voneinander $C_{1-12}$-Alkyl gerade oder verzweigt, $C_{5-8}$-Cycloalkyl, $C_{5-8}$-Cycloalkylmethyl, Benzyl, Phenyl, durch Methyl, Methoxy oder Nitro substituiertes Phenyl oder zusammen eine $C_4$–$C_6$-Alkylenbrücke bedeuten und $R^3$, $R^4$ für Wasserstoff, Fluor, Chlor, Methyl, Methoxy, Trifluormethyl oder Nitro stehen, während sich die Diselenobrücke in ortho-, meta- oder para-Stellung befindet.

Daneben sind Verbindungen bevorzugt, in denen $R^1$ für Wasserstoff steht und $R^2$ $C_{1-18}$-Alkyl gerade oder verzweigt, $C_{3-8}$-Cycloalkyl, $C_{3-8}$-Cycloalkylmethyl, Benzyl, Phenyl, ein- bis dreimal unabhängig voneinander durch Fluor, Chlor, Brom, Methyl, Methoxy, Hydroxy, Trifluormethyl, Nitro, Dimethylamino, Cyan, Carboxy, Methoxycarbonyl, Ethoxycarbonylethyl bzw. Methylendioxy substituiertes Phenyl bedeutet und $R^3$, $R^4$ für Wasserstoff, Fluor, Chlor, Methyl, Methoxy, Trifluormethyl, Nitro oder zusammen für Methylendioxy stehen und die Diselenobrücke sich in ortho-, meta- oder para-Stellung befindet.
Von dieser Gruppe wiederum sind diejenigen Verbindungen bevorzugt, bei denen sich die Diselenobrücke in ortho-Stellung befindet und in denen $R^3$, $R^4$ gleich oder verschieden sind und unabhängig voneinander Fluor, Chlor, Methyl, Methoxy, Trifluormethyl, Nitro oder zusammen Methylendioxy bedeuten, während $R^2$ einen unsubstituierten oder durch Hydroxy substituierten Phenylrest darstellt.

Besonders bevorzugt sind dabei Verbindungen, in denen $R^3$, $R^4$ gleich oder verschieden sind und unabhängig voneinander Fluor, Chlor, Methyl, Methoxy, Trifluormethyl, Nitro oder zusammen Methylendioxy bedeuten, während $R^2$ einen geraden oder verzweigten Alkylrest darstellt.

Erfindungsgemäße Verbindungen sind beispielsweise:
2,2-Diselenobis-(N-dimethyl-benzamid)
2,2-Diselenobis-(N-diethyl-benzamid)
2,2-Diselenobis-(N-di-n-propyl-benzamid)
2,2-Diselenobis-(N-diisopropyl-benzamid)
2,2-Diselenobis-(N-di-n-butyl-benzamid)
2,2-Diselenobis-(N-di-n-hexyl-benzamid)
2,2-Diselenobis-(N-di-n-octyl-benzamid)
2,2-Diselenobis-(N-n-butyl-N-methyl-benzamid)
2,2-Diselenobis-(N-n-butyl-N-ethyl-benzamid)
2,2-Diselenobis-(N-methyl-N-tert-butyl-benzamid)
2,2-Diselenobis-(N-cyclohexyl-N-methyl-benzamid)
2,2-Diselenobis-(N-benzyl-N-methyl-benzamid)
2,2-Diselenobis-(N-benzyl-N-ethyl-benzamid)

2,2-Diselenobis-(N-methyl-N-phenyl-benzamid)
2,2-Diselenobis-(N-methyl-N-4-methoxyphenyl-benzamid)
2,2-Diselenobis-(N-ethyl-N-3-methylphenyl-benzamid)
2,2-Diselenobis-(N-ethyl-N-2-nitrophenyl-benzamid)
2,2-Diselenobis-(N,N-tetramethylen-benzamid)
2,2-Diselenobis-(N,N-pentamethylen-benzamid)
3,3-Diselenobis-(N-dimethyl-benzamid)
3,3-Diselenobis-(N-diisopropyl-benzamid)
3,3-Diselenobis-(N-benzyl-N-methyl-benzamid)
3,3-Diselenobis-(N-methyl-N-phenyl-benzamid)
3,3-Diselenobis-(N,N-pentamethylen-benzamid)
4,4-Diselenobis-(N-dimethyl-benzamid)
4,4-Diselenobis-(N-diethyl-benzamid)
4,4-Diselenobis-(N-diisopropyl-benzamid)
4,4-Diselenobis-(N-benzy-N-methyl-benzamid)
4,4-Diselenobis-(N-methyl-N-phenyl-benzamid)
3,3-Diselenobis-(N-methyl-benzamid)
3,3-Diselenobis-(N-ethyl-benzamid)
3,3-Diselenobis-(N-isopropyl-benzamid)
3,3-Diselenobis-(N-tert-butyl-benzamid)
4,4-Diselenobis-(N-methyl-benzamid)
4,4-Diselenobis-(N-ethyl-benzamid)
4,4-Diselenobis-(N-propyl-benzamid)
4,4-Diselenobis-(N-isopropyl-benzamid)
4,4-Diselenobis (N-tert-butyl-benzamid)
4,4-Diselenobis-(N-benzyl-benzamid)
4,4-Diselenobis-[N-(4-hydroxyphenyl)-benzamid]
4,4-Diselenobis-[N-(2-hydroxy-5-methylphenyl)-benzamid]
4,4-Diselenobis-[N-(4-chlorphenyl)-benzamid]
4,4-Diselenobis-[N-(3-chlor-2-methoxyphenyl)-benzamid]
4,4-Diselenobis-[N-(4-dimethylaminophenyl)-benzamid]
4,4-Diselenobis-[N-(2-methoxy-5-nitrophenyl)-benzamid]
4,4-Diselenobis-[N-(4-cyanphenyl)-benzamid]
4,4-Diselenobis-[N-(2-cyanphenyl)-benzamid]
4,4-Diselenobis-[N-(4-fluorphenyl)-benzamid]
4,4-Diselenobis-[N-(2-trifluormethylphenyl)-benzamid]
4,4-Diselenobis-[N-(3,4-methylendioxyphenyl)-benzamid]
2,2-Diselenobis-(N-phenyl-benzamid)
2,2-Diselenobis-[N-phenyl-(4-fluorbenzamid)]
2,2-Diselenobis-[N-phenyl-(4-chlorbenzamid)]
2,2-Diselenobis-[N-phenyl-(4-methylbenzamid)]
2,2-Diselenobis-[N-phenyl-(3-methoxybenzamid)]
2,2-Diselenobis-[N-phenyl-(4-trifluormethylbenzamid)]
2,2-Diselenobis-[N-phenyl-(5-chlorbenzamid)]
2,2-Diselenobis-[N-phenyl-(3-nitrobenzamid)]
2,2-Diselenobis-[N-phenyl-(3,4-methylendioxybenzamid)]
2,2-Diselenobis-[N-(4-nitrophenyl)-benzamid]
2,2-Diselenobis-[N-(4-fluorphenyl)-benzamid]
2,2-Diselenobis-[N-(4-cyanphenyl)-benzamid]
2,2-Diselenobis-[N-(4-methoxycarbonylmethylphenyl)-benzamid]
2,2-Diselenobis- N-[4-(1-ethoxycarbonylethyl)-phenyl]-benzamid
2,2-Diselenobis-[N-(4-trifluormethylphenyl)-benzamid]
2,2-Diselenobis-[N-(4-methylphenyl)-benzamid]
2,2-Diselenobis-[N-(4-methoxyphenyl)-benzamid]
2,2-Diselenobis-[N-(4-chlorphenyl)-benzamid]
2,2-Diselenobis-[N-(4-dimethylaminophenyl)-benzamid]
2,2-Diselenobis-[N-(2-hydroxyphenyl)-benzamid]
2,2-Diselenobis-[N-(2-methoxyphenyl)-benzamid]
2,2-Diselenobis-[N-(2-methoxycarbonylphenyl)-benzamid]
2,2-Diselenobis-[N-(2-carboxyphenyl)-benzamid]
2,2-Diselenobis-[N-(3-hydroxyphenyl)-benzamid]
2,2-Diselenobis-[N-(4-hydroxyphenyl)-benzamid]
2,2-Diselenobis-[N-(2-nitrophenyl)-benzamid]
2,2-Diselenobis-[N-(2-chlorphenyl)-benzamid]
2,2-Diselenobis-[N-(3,4-dimethoxyphenyl)-benzamid]

2,2-Diselenobis-[N-(3,4-methylendioxyphenyl)-benzamid]
2,2-Diselenobis-(N-benzyl-benzamid)
2,2-Diselenobis-[N-(4-phenylbutyl)-benzamid]
2,2-Diselenobis-(N-cyclohexyl-benzamid)
2,2-Diselenobis-(N-cyclohexylmethyl-benzamid)
2,2-Diselenobis-(N-methyl-benzamid)
2,2-Diselenobis-(N-ethyl-benzamid)
2,2-Diselenobis-(N-propyl-benzamid)
2,2-Diselenobis-(N-isopropyl-benzamid)
2,2-Diselenobis-(N-n-butyl-benzamid)
2,2-Diselenobis-(N-tert-butyl-benzamid)
2,2-Diselenobis-(N-hexyl-benzamid)
2,2-Diselenobis-(N-octyl-benzamid)
2,2-Diselenobis-(N-dodecyl-benzamid)
2,2-Diselenobis-(N-hexadecyl-benzamid)
2,2-Diselenobis-(N-octadecyl-benzamid)

Die erfindungsgemäßen Substanzen weisen glutathionperoxidaseartige Eigenschaften auf und sind damit in der Lage, dieses Enzym zu ersetzen und auf diese Weise im Zusammenwirken mit einem Mercaptan die schädigende Wirkung aktiver Sauerstoffmetaboliten zu verhindern.

Die selenabhängige Glutathion(GSH)-Peroxidase (Px) katalysiert die Reduktion von $H_2O_2$ und organischen Hydroperoxiden.

$$2\ GSH + H_2O_2 \xrightarrow{GSH-Px} GSSG$$

$$2\ GSH + ROOH \xrightarrow{GSH-Px} GSSG + ROH + H_2O$$

Das selenhaltige Enzym schützt die Zellen gegen Peroxidation und spielt eine wichtige Rolle in der Modulierung des Arachidonsäure-Stoffwechsels (C.C. Reddy, E.J. Massaro, Fundam. and Appl. Toxicology (3), 9–10 (1983), S. 431–436 und L. Flohé in Free Radicals in Biology, Vol. V., Edited by W.A. Pryor 1982 Academic Press, S. 223–254). Die Glutathion-Peroxidase spielt bei allen Erkrankungen eine Rolle, bei denen es zu einer Zellschädigung des betreffenden Gewebes und schließlich zur Nekrose aufgrund einer vermehrten Bildung von aktiven Sauerstoffmetaboliten in Form von Peroxiden (z.B. Lipidperoxide und Wasserstoffperoxid) kommt. Dieser sogenannte "oxidative Streß" wird z.B. beobachtet bei Lebererkrankungen – induziert durch entzündliche oder autoimmunologische Reaktionen, durch Alkohol oder durch Medikamente – aber auch bei anderen Erkrankungen, wie z.B. Herzinfarkt. Es ist bekannt, daß nach einem Herzinfarkt in das geschädigte Gebiet Leukozyten einwandern und der Zelluntergang von einer vermehrten Freisetzung der genannten aktiven Sauerstoffmetaboliten begleitet ist. Dies führt schließlich zu einem fortschreitenden Gewebeabbau.

In solchen Fällen ist das hierfür wichtige und natürlicherweise vorhandene Schutzsystem, bestehend aus verschiedenen, Peroxide und aktiven Sauerstoff abbauenden Enzymen, überfordert. Hierzu gehören Superoxiddismutase, Katalase und besonders das Glutathion-Redox-System mit der dazugehörenden Enzymkomponente Glutathion-Peroxidase. Gerade diesem letzteren Prinzip kommt eine große Bedeutung zu, da es sowohl organische Peroxide als auch Wasserstoffperoxid entgiften kann. Es ist belegt, daß dieses System für die intakte Leberfunktion eine große Rolle spielt (Wendel et al.: Biochemical Pharmacology, Vol. 31, S. 361 (1982)) und z.B. das Ausmaß eines experimentellen Leberschadens gerade von diesem System abhängt, d.h. von dem Gehalt der Leber an Glutathion einerseits und von der Aktivität des Enzyms Glutathion-Peroxidase andererseits. Im Verlauf einer allgemeinen Entzündung wird dieser Leberschutzmechanismus stark reduziert (Bragt et al., Agents and Actions, Supp. 17, S. 214 (1980)), wodurch die Leber einen stark erhöhten "oxidativen Streß" erleidet.

Eine sehr wichtige Rolle spielen die reaktiven Sauerstoffmetaboliten als Mediatoren von Entzündungen. Sie scheinen sowohl bei Leucotaxis, Gefäßdurchlässigkeit, Bindegewebsschäden und Immunkomplex/Komplement-induzierten Effekten mitzuwirken als auch bei Schäden, wie sie durch Wiedereinströmen in ischämischen Bereichen entstehen (L. Flohé et al., in The Pharmacology of Inflammation, ed. I.L. Bonta et al., Handbook of Inflammation, Vol. 5, Elsevier, Amsterdam, S. 255–270).

Auch die Schäden nach ionisierender Bestrahlung sind auf die Bildung von Radikalen und aktiven Sauerstoffmetaboliten zurückzuführen. Ein Weg für die chemische Zytoprotektion besteht somit in der Stärkung des Glutathion/Glutathionperoxidase-Systems (H. Rink in: "Glutathione", Proceedings of the 16th Conference of the German Society of Biological Chemistry 1973, edited by Flohé, Benöhr, Sies, Walter and Wendel, Seite 206).

Die Messung der glutathionperoxidaseartigen Aktivität erfolgte nach der Methode von A. Wendel (A. Wendel, Methods in Enzymology Vol. 77, 325–333 (1981)). Gemessen wird in diesem Versuch das Verschwinden von GSH in Gegenwart von t-Butylhydroperoxid. Es wurde nun überraschend gefunden, daß die erfindungsgemäßen Verbindungen der Formel I glutathionperoxidaseartige Wirkung besitzen.

Glutathionperoxidaseartige Wirkung

Bei in vitro-Untersuchungen wurde die Katalyse des Peroxidaseabbaus geprüft. Dabei wurde festgestellt, daß die erfindungsgemäßen Verbindungen die Glutathion-Peroxidase ersetzen können.

ROOH —Erfindungsgemäße Verbindungen→ ROH

$H_2O_2$ $H_2O$

RSH RSSR

Die katalytische Aktivität wird in GSH-Px Einheiten ausgedrückt. Als Referenzsubstanz diente Ebselen, 2-Phenyl-1,2-benzisoselenazol-3(2H)-on (A. Wendel, M. Fansel, H. Safayhi, G. Tiegs, R. Otter, Biochem. Pharmac. 33, 3241, 1984).
Zum besseren Vergleich wurde die Aktivität von Ebselen als 100% angesehen und die Aktivität der erfindungsgemäßen Verbindungen auf die von Ebselen bezogen.
Ebselen wurde in einer Konzentration von 30 µMol eingesetzt, als Lösungsvermittler diente DMF. Die Diselenide wurden in einer Konzentration von 15 µMol eingesetzt (Dimethylformamid), da in den Diseleniden 2 Selenatome/Mol vorhanden sind.

| Substanz | Katalytische Aktivität (in %) |
|---|---|
| Ebselen | 100 |
| 2,2-Diselenobis-(N-dimethyl-benzamid) | 180 |
| 2,2-Diselenobis-(N-diisopropyl-benzamid) | 200 |
| 2,2-Diselenobis-(N-pentamethylen-benzamid) | 125 |
| 2,2-Diselenobis-(N-methyl-N-phenyl-benzamid) | 225 |
| 3,3-Diselenobis-(N-dimethyl-benzamid) | 220 |
| 2,2-Diselenobis-(benzamid) | 105 |
| 2,2-Diselenobis-(N-benzyl-benzamid) | 105 |
| 2,2-Diselenobis-[N-(trifluormethylphenyl)-benzamid] | 100 |
| 2,2-Diselenobis-(N-cyclohexylmethyl-benzamid) | 90 |
| 2,2-Diselenobis-[N-(3-hydroxyphenyl)-benzamid] | 115 |
| 2,2-Diselenobis-[N-(3,5-dichlorphenyl)-benzamid] | 85 |
| 2,2-Diselenobis-(N-methyl-benzamid) | 110 |

Die Herstellung der erfindungsgemäßen Verbindungen ist durch ein Verfahren gekennzeichnet, bei dem Diselenobisbenzoesäuren der allgemeinen Formel II

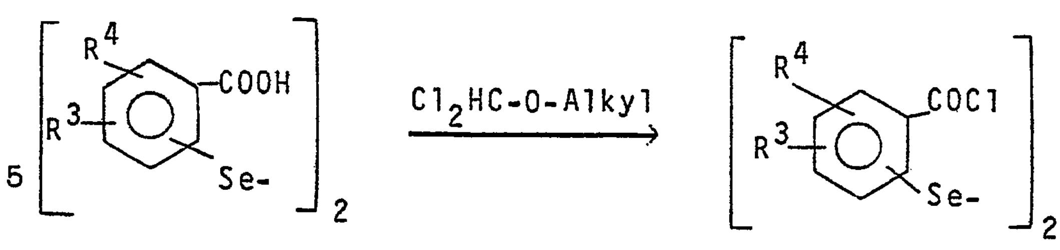


II III

worin R³, R⁴ die in Formel I angegebenen Bedeutungen haben und die Diselenobrücke sich in ortho-, meta- oder para-Stellung befindet, in einem geeigneten Lösungsmittel mit Dichlormethylalkylethern, wie z.B. Dichlormethylmethylether, Dichlormethylbutylether in Gegenwart von Zinkchlorid innerhalb von 72 Stunden bei Raumtemperatur in die entsprechenden Säurechloride der allgemeinen Formel III umgesetzt wer-

den, die nach Isolierung durch Umsetzung mit Aminen der allgemeinen Formel IV

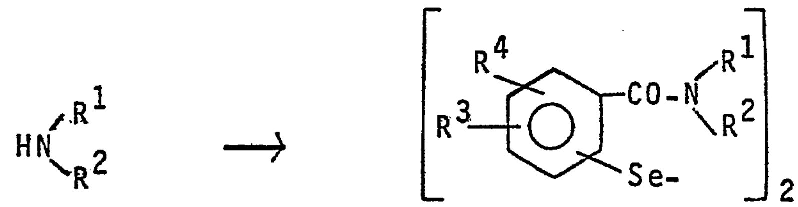

IV I

worin $R^1$, $R^2$ die in Formel I angegebenen Bedeutungen haben, nach an sich bekannten Methoden in die Verbindungen der Formel I übergeführt werden. Die als Ausgangssubstanzen verwendeten Diseleno-bis-benzoesäuren sind nach verschiedenen Methoden zugänglich.

<u>Methode 1</u> (A. Ruwet, M. Renson, Bull. Soc. Chim. Belg. <u>75</u>, 157–163 (1966))

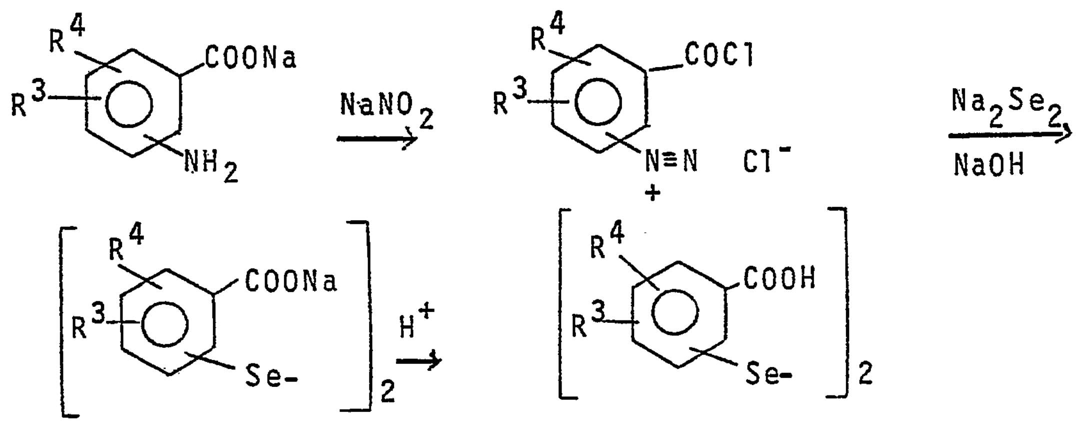


Die Ausbeuten dieser Reaktion sind gut (90% d.Th.) soweit es sich um 2-Aminobenzosäuren handelt und zufriedenstellend für substituierte 2-Aminobenzoesäuren. Bei den 3- und 4-Aminobenzoesäuren werden nur sehr geringe Ausbeuten erhalten.

<u>Methode 2</u> (W.R. Gaythwaite, J. Kenyon, H. Phillips, J. Chem. Soc. 2280 (1928) u. J.W. Baker, E.F.C. Banett, W.T. Tweed, J. Chem. Soc. 2831 (1952))

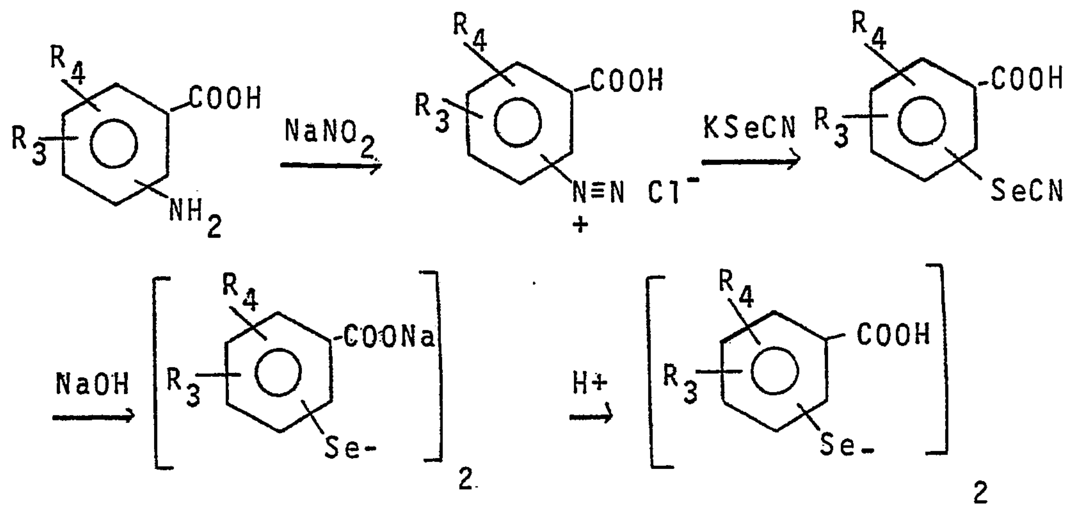

Nach dieser Methode lassen sich auch 3,3- und 4,4-Diselenobenzoesäuren in besseren Ausbeuten herstellen.

Die zur Umsetzung verwendeten Amine sind bekannte Verbindungen, wie z.B. Dimethylamin, Diethylamin, Dipropylamin, Diisopropylamin, Di-n-butylamin, Diisobutylamin, Di-n-hexylamin, Di-n-heptylamin, Di-n-octylamin, Di-n-decylamin, Di-n-dodecylamin, N-n-Butyl-methylamin, N-n-Butyl-ethylamin, N-tert-Butyl-methylamin, N-Cyclohexyl-methylamin, N-Cyclopentyl-methylamin, N-Cyclooctyl-methylamin, N-Benzylmethylamin, N-Ethylbenzylamin, N-Methylanilin, N-Ethylanilin, N-Methyl-p-anisidin, N-Ethyl-m-toluidin, N-Ethyl-2-nitroanilin, Pyrrolidin, Piperidin, Methylamin, Ethylamin, Propylamin, Isopropylamin, tert-Butylamin, Cyclopropylamin, Cyclobutylamin, Cyclopentylamin, Cyclohexylamin, Cyclooctylamin, Cyclopropylmethylamin, Cyclohexylmethylamin, Cycloheptylmethylamin, Cyclooctylmethylamin, n-Pentylamin, n-Hexylamin, n-Heptylamin, n-Octylamin, n-Decylamin, n-Dodecylamin, n-Hexadecylamin, n-Octadecylamin, Benzylamin, 4-Aminophenol, 3-Aminophenol, 2-Aminophenol, 2-Amino-4-methylphenol, 4-Amino-3-methylphenol, 2-Amino-5-methylphenol, 4-Amino-2,6-dichlorphenol, 4-Amino-2,6-dimethylphenol, 4-Chloranilin, 2-Chloranilin, 4-Bromanilin, 3-Chlor-2-methoxyanilin, 2-Chlor-5-methoxyanilin, N,N-Dimethyl-p-phenylendiamin, 4-Methoxy-2-methylanilin, 2-Methoxy-5-nitroanilin, 4-Cyananilin, 3-Cyananilin, 2-Cyananilin, 2-Cyan-4-nitroanilin, 4-Fluoranilin, 2-Fluoranilin, 2-Fluor-5-nitroanilin, 4-Trifluormethylanilin, 2-Trifluormethylanilin, 3,4-Methylendioxyanilin.

Eine weitere Möglichkeit zur Herstellung der erfindungsgemäßen Verbindungen besteht in der Umsetzung von Benzisoselenazolonen der Formel V, worin $R^2$, $R^3$, $R^4$ die in Formel I angegebenen Bedeutungen haben, die nach den Vorschriften von DE-OS 3 027 073 = USP 4 352 799 bzw. DE-OS 3 027 075 = USP 4 418 069 erhalten werden, mit einer annähernd äquimolekularen Menge eines Mercaptans, wie z.B. Ethylmercaptan in einem geeigneten organischen Lösungsmittel bei Raumtemperatur zu Zwischenverbindungen der Formel VI

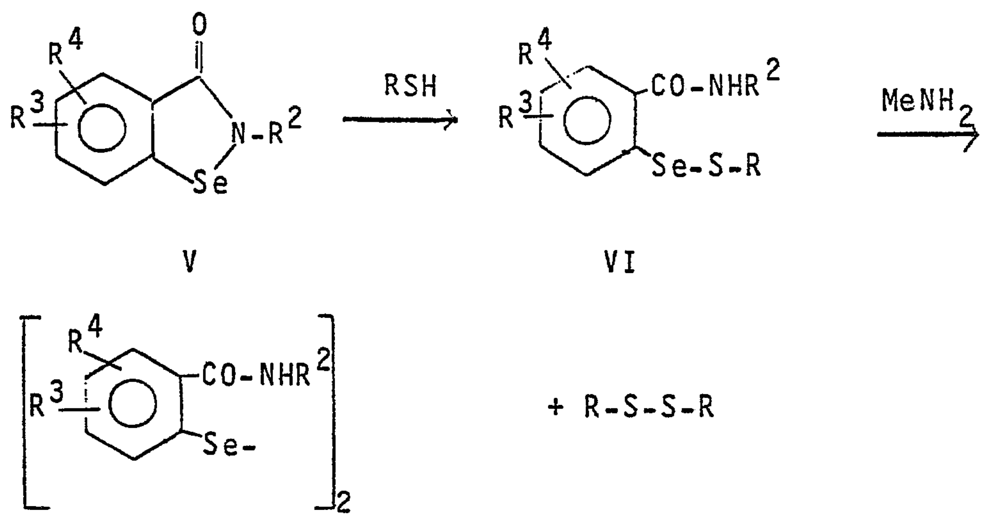


V

VI

I

die in Gegenwart eines Amins, wie z.B. Methylamin, leicht unter Disproportionierung in die erfindungsgemäßen Verbindungen der Formel I umgewandelt werden.

Die vorliegende Erfindung betrifft ebenfalls pharmazeutische Präparate, welche Verbindungen der Formel I enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Substanzen liegt üblicherweise zwischen 10 und 1000 mg pro Tag, vorzugsweise zwischen 30 und 300 mg pro Tag und kann in einer Dosis oder mehreren Teildosen, vorzugsweise in zwei bis drei Teildosen pro Tag, verabreicht werden.

Die Herstellung der erfindungsgemäßen Verbindungen wird durch die folgenden Beispiele näher erläutert. Die angegebenen Schmelzpunkte wurden mit einem Büchi 510-Schmelzpunktbestimmungsapparat gemessen und sind mit °C angegeben und nicht korrigiert.

Allgemeine Vorschrift für die Herstellung der Diselenobisbenzoesäurechloride am Beispiel der 2,2-Diselenobis-benzoesäure.

20 g (50 mMol) 2,2-Diselenobis-benzoesäure werden in 400 ml trockenem Dichlormethan suspendiert.

Dieser Mischung werden 45 ml (500 mMol) Dichlormethylmethylether sowie 1 g trockenes Zinkchlorid zugegeben. Das Reaktionsgemisch wird 72 Stunden bei Raumtemperatur weitergerührt. Nach Abfiltrieren des Rückstandes wird die Lösung eingeengt. Das Säurechlorid wird aus Toluol umkristallisiert. Man erhält 6 g reines Produkt. Durch partielles Einengen der Mutterlauge werden noch weitere 4,6 g erhalten.
Ausbeute: 10,6 g (48,5% d.Th.) Fp. 170°C

Beispiel 1

2,2-Diselenobis-(N-dimethyl-benzamid).

4,0 g (9,15 mMol) 2,2-Diselenobis-benzoesäurechlorid werden in 50 ml Diisopropylether gelöst. Diese Lösung wird einer 40%igen Lösung Dimethylamin in Wasser (10 ml) unter Rühren zugetropft. Nach 15 Minuten Rühren gibt man 100 ml Wasser zu dem Gemisch, filtriert den Niederschlag ab und wäscht mit 50 ml Wasser nach. Der Niederschlag wird getrocknet und aus Toluol/Hexan umkristallisiert.
Ausbeute: 3,1 g (75% d.Th.) Fp. 173–174°C

Beispiel 2

2,2-Diselenobis-(N-diisopropyl-benzamid).

4,0 g (9,15 mMol) 2,2-Diselenobis-benzoesäurechlorid werden in 50 ml Pyridin gelöst. Diese Lösung wird einer Lösung von 2,0 g (19,8 mMol) Diisopropylamin in 20 ml Pyridin zugetropft. Man rührt noch 15 Minuten weiter und gießt die Lösung auf ein Gemisch von Eis-Wasser-verdünnter Salzsäure (ca. 250 ml). Der Niederschlag wird abfiltriert, mit Wasser gewaschen und getrocknet.
Ausbeute: 4,1 g (93,3% d.Th.) Fp. 156–158°C

Beispiel 3

2,2-Diselenobis-(N,N-pentamethylen-benzamid).

Analog Beispiel 2 aus:
4,0 g (9,15 mMol) 2,2-Diselenobis-benzoesäurechlorid
2,0 g (23,5 mMol) Piperidin
Ausbeute: 3,8 g (77,7% d.Th.) Fp. 159–160°C

Beispiel 4

2,2-Diselenobis-(N-benzyl-N-methyl-benzamid).

Analog Beispiel 2 aus:
4,0 g (9,15 mMol) 2,2-Diselenobis-benzoesäurechlorid
2,4 g (19,8 mMol) N-Benzylmethylamin
Ausbeute: 2,9 g (52,3% d.Th.) Fp. 139–140°C

Beispiel 5

2,2-Diselenobis-(N-methyl-N-phenyl-benzamid).

Analog Beispiel 2 aus:
4,0 g (9,15 mMol) 2,2-Diselenobis-benzoesäurechlorid
2,2 g (20,6 mMol) N-Methylanilin
Ausbeute: 3,8 g (71,85% d.Th.) Fp. 83°C

Beispiel 6

3,3-Diselenobis-(N-dimethyl-benzamid).

Analog Beispiel 1 aus:
5,46 g (12,49 mMol) 3,3-Diselenobis-benzoesäurechlorid
10 ml 40%ige wäßrige Dimethylaminlösung
Ausbeute: 2,8 g (49,4% d.Th.) Fp. 165–167°C

Beispiel 7

4,4-Diselenobis-(N-dimethyl-benzamid).

Analog Beispiel 1 aus:
4,0 g (9,15 mMol) 4,4-Diselenobis-benzoesäurechlorid
10 ml 40%ige wäßrige Dimethylaminlösung
Ausbeute: 3,2 g (77% d.Th.) Fp. 180–182°C

Beispiel 8

3,3-Diselenobis-(N-phenyl-benzamid).

2,0 g (4,6 mMol) 3,3-Diselenobis-benzoesäurechlorid werden in 20 ml trockenem Pyridin gelöst. Zu dieser Lösung werden 0,9 g Anilin (9,6 mMol) in 10 ml Pyridin zugetropft. Die Lösung wird 15 Minuten weitergerührt und anschließend in ein Eis-Wasser-verd. Salzsäure-Gemisch eingetragen. Der Niederschlag wird abfiltriert, mit Wasser gewaschen und getrocknet.
Nach Umkristallisieren aus Dimethylformamid erhält man
1,72 g (68% d.Th.) Fp. 209–212°C

Beispiel 9

3,3-Diselenobis-(N-methyl-benzamid).

Analog Beispiel 1 aus:
2 g (4,6 mMol) 3,3-Diselenobis-benzoesäurechlorid
5 ml 40%ige wäßrige Methylaminlösung
Ausbeute: 1 g (55% d.Th.) Fp. 172°C

Beispiel 10

3,3-Diselenobis-[N-(4-hydroxyphenyl)-benzamid].

Analog Beispiel 8 aus:
2 g (4,6 mMol) 3,3-Diselenobis-benzoesäurechlorid
1,0 g (9,17 mMol) 4-Aminophenol
Ausbeute: 0,93 g (35% d.Th.) Fp. 268–270°C

Beispiel 11

4,4-Diselenobis-(N-phenyl-benzamid).

Analog Beispiel 8 aus:
2 g 4,4-Diselenobis-benzoesäurechlorid
0,8 g Anilin
Ausbeute: 1,43 g (57% d.Th.) Fp. 271–274°C

Beispiel 12

4,4-Diselenobis-[N-(4-fluorphenyl)-benzamid].

Analog Beispiel 8 aus:
2 g 4,4-Diselenobis-benzoesäurechlorid
1,0 g 4-Fluoranilin
Ausbeute: 1,51 g (56,5% d.Th.) Fp. 265–268°C

Beispiel 13

4,4-Diselenobis-(N-tert-butyl-benzamid).

Analog Beispiel 8 aus:
2 g 4,4-Diselenobis-benzoesäurechlorid
0,8 g tert-Butylamin
Ausbeute: 1 g (44,3% d.Th.) Fp. 212–215°C

Beispiel 14

4,4-Diselenobis-(N-methyl-benzamid).

Analog Beispiel 8 aus:
2 g 4,4-Diselenobis-benzoesäurechlorid
5 ml 40%ige wäßrige Methylaminlösung
Ausbeute: 0,8 g (44% d.Th.) Fp.

Beispiel 15

2,2-Diselenobis-(N-diphenyl-benzamid).

Analog Beispiel 2 aus:
4,0 g 2,2-Diselenobis-benzoesäurechlorid
3,3 g Diphenylamin
Ausbeute: 3,9 g (54,5% d.Th.) Fp. 208–210°C

Beispiel 16

2,2-Diselenobis-(N-benzyl-N-phenyl-benzamid).

Analog Beispiel 2 aus:
4,0 g 2,2-Diselenobis-benzoesäurechlorid
3,4 g N-Benzylanilin
Ausbeute: 3,2 g (47,9% d.Th.) Fp. 242–244°C

Beispiel 17

2,2-Diselenobis-[N-(4-methoxyphenyl)-N-methyl-benzamid].

Analog Beispiel 2 aus:
4,0 g 2,2-Diselenobis-benzoesäurechlorid
3,3 g 4-Methoxy-N-methylanilin
Ausbeute: 2,9 g (49,7% d.Th.) Fp. 218–220°C

Beispiel 18

2,2-Diselenobis-[N-Ethyl-(4-fluorphenyl)-benzamid].

Analog Beispiel 2 aus:
4,0 g 2,2-Diselenobis-benzoesäurechlorid
3,3 g N-Ethyl-4-fluoranilin
Ausbeute: 3,5 g (57,8% d.Th.) Fp. 132–133°C

Beispiel 19

2,2-Diselenobis-[N-ethyl-(3-methylphenyl)-benzamid].

Analog Beispiel 2 aus:
4,0 g 2,2-Diselenobis-benzoesäurechlorid
3,3 g N-Ethyl-m-toluidin
Ausbeute: 3,2 g (55% d.Th.) Fp. 120–121°C

Beispiel 20

2,2-Diselenobis-[N-methyl-(3-methylphenyl)-benzamid].

Analog Beispiel 2 aus:
4,0 g 2,2-Diselenobis-benzoesäurechlorid
3,4 g N-Methyl-m-toluidin
Ausbeute: 3,0 g (54,1% d.Th.) Fp. 113–115°C

Beispiel 21

2,2-Diselenobis-[N-methyl-(2-methoxycarbonylphenyl)-benzamid].

Analog Beispiel 2 aus:
4,0 g 2,2-Diselenobis-benzoesäurechlorid
3,9 g N-Methyl-anthranilsäuremethylester
Ausbeute: 2,8 g (44,1% d.Th.) Fp. 104–105°C

Allgemeine Vorschrift

5 mMol eines 2-substituierten 1,2-Benzisoselenazol-3(2H)-ons werden in ca. 200 ml Methanol suspendiert und mit 5,5 mMol eines Mercaptans (z.B. Ethylmercaptan) versetzt. Nach wenigen Minuten (5–15 Min.) entsteht eine klare Lösung. Mittels Dünnschichtchromatographie wird geprüft, ob sich das 1,2-Benzisoselenazol-3(2H)-on vollständig umgesetzt hat. Sobald dies der Fall ist, werden 2 ml einer 33%igen wäßrigen Lösung von Methylamin zugegeben. Das Reaktionsgemisch wird mindestens eine Stunde gerührt. Der entstandene Niederschlag wird abgesaugt, mit Methanol und schließlich mit Ether gewaschen. In einigen Fällen wird noch in Methylenchlorid ausgerührt. Wenn notwendig kann das so erhaltene Diselenid aus einem geeigneten Lösungsmittel umkristallisiert werden.
* In einigen Fällen kristallisiert das Zwischenprodukt nach wenigen Minuten wieder aus. In diesen Fällen wird dem Reaktionsgemisch soviel Dimethylformamid zugegeben, bis wieder eine klare Lösung erhalten wird.

Beispiel 22

2,2-Diselenobis-[N-(4-nitrophenyl)-benzamid].

Analog der allgemeinen Vorschrift aus:
1,0 g (3,13 mMol) 2-(4-Nitrophenyl)-1,2-benzisoselenazol-3(2H)-on
0,5 ml Ethylmercaptan
1,5 ml 33%iges Methylamin
in 100 ml Methanol
Ausbeute: 0,65 g (65% d.Th.) Fp 243–245°C

Beispiel 23

2,2-Diselenobis-[N-(3-hydroxyphenyl)-benzamid].

Analog Beispiel 22 aus:
5 g 2-(3-Hydroxyphenyl)-1,2-benzisoselenazol-3(2H)-on
1,3 ml Ethylmercaptan
4,3 ml 33%iges Methylamin
in 250 ml Methanol
Ausbeute: 1,2 g (12% d.Th.) Fp. 285–286°C

Beispiel 24

2,2-Diselenobis-[N-(4-hydroxyphenyl)-benzamid].

Analog Beispiel 22 aus:
2,5 g 2-(4-Hydroxyphenyl)-1,2-benzisoselenazol-3(2H)-on
2,0 ml 33%iges Methylamin
0,65 ml Ethylmercaptan
in 100 ml Methanol
Ausbeute: 1,15 g (46% d.Th.) Fp. 282–284°C

Beispiel 25

2,2-Diselenobis-[N-(2-hydroxyphenyl)-benzamid].

Analog Beispiel 22 aus:
3 g 2-(2-Hydroxyphenyl)-1,2-benzisoselenazol-3(2H)-on
0,78 ml Ethylmercaptan
3 ml 33%iges Methylamin
in 100 ml Methanol
Ausbeute: 0,5 g (8,3% d.Th.) Fp. 235–238°C

Beispiel 26

2,2-Diselenobis-(N-cyclohexylmethyl-benzamid).

Analog Beispiel 22 aus:
2,5 g 2-Cyclohexylmethyl-1,2-benzisoselenazol-3(2H)-on
0,53 ml Ethylmercaptan
2,1 ml 33%iges Methylamin
in 100 ml Methanol
Ausbeute: 2,15 g (86% d.Th.) Fp. 227–229°C

Beispiel 27

2,2-Diselenobis-[N-(2-trifluormethyl)-benzamid].

Analog Beispiel 22 aus:
1 g 2-(2-Trifluormethylphenyl)-1,2-benzisoselenazol-3(2H)-on
0,215 ml Ethylmercaptan
0,73 ml 33%iges Methylamin
in 50 ml Methanol
Ausbeute: 0,508 g (51,2% d.Th.) Fp. 235–236°C

Beispiel 28

2,2-Diselenobis- N-[4-(1-ethoxycarbonylethyl)-phenyl]-benzamid.

Analog Beispiel 22 aus:
0,7 g 2-[4-(1-Ethoxycarbonylethyl)-phenyl]-1,2-benzisoselenazol-3(2H)-on
0,14 ml Ethylmercaptan
0,68 ml 33%iges Methylamin
in 50 ml Methanol
Ausbeute: 0,1 g (14,3% d.Th.) Fp. 179–181°C

Beispiel 29

2,2-Diselenobis-[N-(3,4-methylendioxyphenyl)-benzamid].

Analog Beispiel 22 aus:
0,82 g 2-(3,4-Methylendioxyphenyl)-1,2-benzisoselenazol-3(2H)-on
0,19 ml Ethylmercaptan
0,94 ml 33%iges Methylamin
in 50 ml Methanol
Ausbeute: 0,7 g (85,4% d.Th.) Fp. 270–272°C

Beispiel 30

2,2-Diselenobis-[N-(4-dimethylaminophenyl)-benzamid].

Analog Beispiel 22 aus:
2 g 2-(4-Dimethylaminophenyl)-1,2-benzisoselenazol-3(2H)-on
0,46 ml Ethylmercaptan
1,18 ml 33%iges Methylamin
in 100 ml Methanol
Ausbeute: 1 g (49,8% d.Th.) Fp. 258–260°C

Beispiel 31

2,2-Diselenobis-[N-(4-trifluormethylphenyl)-benzamid].

Analog Beispiel 22 aus:
2 g 2-(4-Trifluormethylphenyl)-1,2-benzisoselenazol-3(2H)-on
0,428 ml Triethylamin
1,45 ml 33%iges Methylamin
in 100 ml Methanol
Ausbeute: 0,87 g (43,8% d.Th.) Fp. 273°C

Beispiel 32

2,2-Diselenobis-[N-(4-methylphenyl)-benzamid].

Analog Beispiel 22 aus:
2 g 2-(4-Methylphenyl)-1,2-benzisoselenazol-3(2H)-on
0,5 ml Ethylmercaptan
1,73 ml 33%iges Methylamin
in 100 ml Methanol
Ausbeute: 1,44 g (71,8% d.Th.) Fp. 255°C

Beispiel 33

2,2-Diselenobis-(N-benzyl-benzamid).

Analog Beispiel 22 aus:
2 g 2-Benzyl-1,2-benzisoselenazol-3(2H)-on
0,5 ml Ethylmercaptan
1,73 ml 33%iges Methylamin
in 100 ml Methanol
Ausbeute: 1,37 g (68,4% d.Th.) Fp. 196–198°C

Beispiel 34

2,2-Diselenobis-[N-(4-methoxyphenyl)-benzamid].

Analog Beispiel 22 aus:
2g 2-(4-Methoxyphenyl)-1,2-benzisoselenazol-3(2H)-on
0,48 ml Ethylmercaptan
1,6 ml 33%iges Methylamin
in 150 ml Methanol
Ausbeute: 1,26 g (63,2% d.Th.) Fp. 290–292°C

Beispiel 35

2,2-Diselenobis-[N-(4-chlorphenyl)-benzamid].

Analog Beispiel 22 aus:
2 g 2-(4-Chlorphenyl)-1,2-benzisoselenazol-3(2H)-on
0,48 ml Ethylmercaptan
0,53 ml 33%iges Methylamin
in 150 ml Methanol
Ausbeute: 1,45 g (72% d.Th.) Fp. 280–283°C

Beispiel 36

2,2-Diselenobis-[N-(4-phenylbutyl)-benzamid].

Analog Beispiel 22 aus:
2 g 2-(4-Phenylbutyl)-1,2-benzisoselenazol-3(2H)-on
0,45 ml Ethylmercaptan
1,5 ml 33%iges Methylamin
in 150 ml Methanol
Ausbeute: 1,8 g (90% d.Th.) Fp. 180–181°C

Beispiel 37

2,2-Diselenobis-[N-(3,5-dichlorphenyl)-benzamid].

Analog Beispiel 22 aus:
1,7 g 2-(3,5-Dichlorphenyl)-1,2-benzisoselenazol-3(2H)-on
0,4 ml Ethylmercaptan
0,5 ml 33%iges Methylamin
in 200 ml Methanol
Ausbeute: 2,9 g (84% d.Th.) Fp. 255–257°C

Beispiel 38

2,2-Diselenobis-[N-(2-nitrophenyl)-benzamid].

Analog Beispiel 22 aus:
0,5 g 2-(2-Nitrophenyl)-1,2-benzisoselenazol-3(2H)-on
0,12 ml Ethylmercaptan
0,2 ml 33%iges Methylamin
in 50 ml Methanol
Ausbeute: 0,45 g (90% d.Th.) Fp. 225°C

Beispiel 39

2,2-Diselenobis-[N-(2-chlorphenyl)-benzamid].

Analog Beispiel 22 aus:
2 g 2-(2-Chlorphenyl)-1,2-benzisoselenazol-3(2H)-on
0,6 ml Ethylmercaptan
0,7 ml 33%iges Methylamin
in 150 ml Methanol
Ausbeute: 0,8 g (32,2% d.Th.) Fp.

Beispiel 40

2,2-Diselenobis-(N-methyl-benzamid).

Analog Beispiel 22 aus:
1 g 2-Methyl-1,2-benzisoselenazol-3(2H)-on
0,35 ml Ethylmercaptan
1,2 ml 33%iges Methylamin
in 100 ml Methanol
Ausbeute: 0,65 g (63,8% d.Th.) Fp. 277–279°C

Beispiel 41

2,2-Diselenobis-[N-phenyl-(5-methoxy-benzamid)].

Analog Beispiel 22 aus:
1 g 5-Methoxy-2-phenyl-1,2-benzisoselenazol-3(2H)-on
0,25 ml Ethylmercaptan
1 ml 33%iges Methylamin
in 80 ml Methanol
Ausbeute: 0,6 g (60% d.Th.) Fp. 203–204°C

Beispiel 42

2,2-Diselenobis-[N-(4-cyanophenyl)-benzamid].

Analog Beispiel 22 aus:
2 g 2-(2-Cyanophenyl)-1,2-benzisoselenazol-3(2H)-on
0,5 ml Ethylmercaptan
5 ml 33%iges Methylamin
in 150 ml Methanol
Ausbeute: 0,5 g (25% d.Th.) Fp. 268–270°C

Beispiel 43

2,2-Diselenobis-(N-cyclopentyl-benzamid).

Analog Beispiel 22 aus:
0,7 g 2-Cyclopentyl-1,2-benzisoselenazol-3(2H)-on
0,2 ml Ethylmercaptan
3 ml 33%iges Methylamin
in 50 ml Methanol
Ausbeute: 0,46 g (65,7% d.Th.) Fp. 275–280°C

Beispiel 44

2,2-Diselenobis-(N-cyclohexyl-benzamid).

Analog Beispiel 22 aus:
1 g 2-Cyclohexyl-1,2-benzisoselenazol-3(2H)-on
0,25 ml Ethylmercaptan
3 ml 33%iges Methylamin
in 100 ml Methanol
Ausbeute: 0,5 g (50% d.Th.) Fp. 315°C

Beispiel 45

2,2-Diselenobis-[N-(4-fluorphenyl)-benzamid].

Analog Beispiel 22 aus:
1,26 g 2-(4-Fluorphenyl)-1,2-benzisoselenazol-3(2H)-on
0,3 ml Ethylmercaptan
3 ml 33%iges Methylamin
in 100 ml Methanol
Ausbeute: 1,1 g (87,3% d.Th.) Fp. 260°C

Beispiel 46

2,2–Diselenobis-(N-hexyl-benzamid).

Analog Beispiel 22 aus:
2 g 2-Hexyl-1,2-benzisoselenazol-3(2H)-on
0,52 ml Ethylmercaptan
2 ml 33%iges Methylamin
in 150 ml Methanol
Ausbeute: 1,55 g (77,5% d.Th.) Fp. 163–165°C

Beispiel 47

2,2-Diselenobis-(N-tert-butyl-benzamid).

Analog Beispiel 22 aus:
1,34 g 2-tert-Butyl-1,2-benzisoselenazol-3(2H)-on
0,4 ml Ethylmercaptan
1,4 ml 33%iges Methylamin
in 100 ml Methanol
Ausbeute: 0,96 g (71,4% d.Th.) Fp. 241–242°C

Beispiel 48

2,2-Diselenobis-(N-phenyl-benzamid).

Analog Beispiel 22 aus:
2 g 2-Phenyl-1,2-benzisoselenazol-3(2H)-on
0,5 ml Ethylmercaptan
4 ml 33%iges Methylamin
in 100 ml Methanol
Ausbeute: 1,8 g (90% d.Th.) Fp. 263–265°C

Beispiel 49

2,2-Diselenobis-[N-(3,4-dimethoxyphenyl)-benzamid].

Analog Beispiel 22 aus:
1 g 2-(3,4-Dimethoxyphenyl)-1,2-benzisoselenazol-3(2H)-on
0,2 ml Ethylmercaptan
2 ml 33%iges Methylamin
in 150 ml Methanol
Ausbeute: 0,25 g (25% d.Th). Fp. 235°C

Beispiel 50

2,2-Diselenobis-[N-(2-methoxyphenyl)-benzamid].

Analog Beispiel 22 aus:
2 g 2-(2-Methoxyphenyl)-1,2-benzisoselenazol-3(2H)-on
0,5 ml Ethylmercaptan
1,5 ml 33%iges Methylamin
in 100 ml Methanol
Ausbeute: 0,95 g (47,4% d.Th.) Fp. 216–217°C

Beispiel 51

2,2-Diselenobis-[N-phenyl-(3-methoxy-benzamid)].

Analog Beispiel 22 aus:
1 g 7-Methoxy-2-phenyl-1,2-benzisoselenazol-3(2H)-on
0,25 ml Ethylmercaptan
1,0 ml 33%iges Methylamin
in 100 ml Methanol
Ausbeute: 0,31 g (31,6% d.Th.) Fp. 152–155°C

Beispiel 52

2,2-Diselenobis-[N-(2-methoxycarbonylphenyl)-benzamid].

Analog Beispiel 22 aus:
1,66 g 2-(2-Methoxycarbonylphenyl)-1,2-benzisoselenazol-3(2H)-on
0,5 ml Ethylmercaptan
1,5 ml 33%iges Methylamin
in 100 ml Methanol
Ausbeute: 0,55 g (25%) Fp. 230–231°C

Beispiel 53

2,2-Diselenobis-[N-(2-carboxyphenyl)-benzamid].

Analog Beispiel 22 aus:
1,5 g 2-(2-Carboxyphenyl)-1,2-benzisoselenazol-3(2H)-on
0,5 ml Ethylmercaptan
1,5 ml 33%iges Methylamin
in 150 ml Methanol
Ausbeute: 0,49 g (31%) Fp. 298–300°C

Beispiel 54

2,2-Diselenobis-[N-phenyl-(3,4-dimethoxy-benzamid)].

Analog Beispiel 22 aus:
1 g 6,7-Dimethoxy-2-phenyl-1,2-benzisoselenazol-3(2H)-on
0,2 ml Ethylmercaptan
3 ml 33%iges Methylamin
in 200 ml Methanol
Ausbeute: 0,55 g (55% d.Th.) Fp. 235°C

Beispiel 55

2,2-Diselenobis-[N-phenyl-(3-methyl-benzamid)].

Analog Beispiel 22 aus:
2 g 7-Methyl-2-phenyl-1,2-benzisoselenazol-3(2H)-on
0,5 ml Ethylmercaptan
2 ml 33%iges Methylamin
in 100 ml Methanol
Ausbeute: 1,5 g (25% d.Th.) Fp. 290–295°C

Beispiel 56

2,2-Diselenobis-[N-tert-butyl-(3-methoxybenzamid)].

Analog Beispiel 22 aus:
2 g 7-Methoxy-2-tert-butyl-1,2-benzisoselenazol-3(2H)-on
0,5 ml Ethylmercaptan
2 ml 33%iges Methylamin
Ausbeute: 1,1 g (55% d.Th.) Fp. 230–231°C

Beispiel 57

2,2-Diselenobis-[N-methyl-(3-methoxy-benzamid)].

Analog Beispiel 22 aus:
2 g 7-Methoxy-2-methyl-1,2-benzisoselenazol-3(2H)-on
0,5 ml Ethylmercaptan
2 ml 33%iges Methylamin
in 100 ml Methanol
Ausbeute: 1,7 g (65% d.Th.) Fp. 216–217°C

**Patentansprüche für die Vertragsstaaten BE, CH, DE, FR, GB, IT, LI, LU, NL, SE**

1. Diselenobis-benzoesäureamide der allgemeinen Formel I

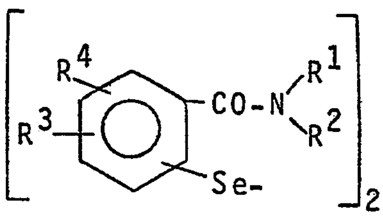

I

worin
$R^1$, $R^2$ gleich oder verschieden sind, mit der Maßgabe, daß mindestens ein Rest ungleich Wasserstoff sein muß, unabhängig voneinander Wasserstoff, $C_{1-18}$-Alkyl gerade oder verzweigt, $C_{3-8}$-Cycloalkyl, $C_{3-8}$-Cycloalkylmethyl, Phenylalkyl, Phenyl, ein- bis dreimal unabhängig voneinander durch Halogen, $C_{1-4}$-Alkyl, $C_{1-4}$-Alkoxy, Hydroxy, Trifluormethyl, Nitro, Di-($C_{1-4}$-alkyl)-amino, Cyan, Carboxy, Alkoxycarbonyl, Alkoxycarbonylalkyl bzw. Methylendioxy substituiertes Phenyl bedeuten oder zusammen eine $C_{4-6}$-Alkylenbrücke bilden und

$R^{33}$, $R^4$ gleich oder verschieden sind und unabhängig voneinander für Wasserstoff, Halogen, $C_{1-4}$-Alkyl, $C_{1-4}$-Alkoxy, Trifluormethyl, Nitro oder zusammen für Methylendioxy stehen und die Diselenobrücke in beiden Ringen in ortho-, meta- oder para-Stellung zur Carbamidgruppe steht.

2. Diselenobis-benzoesäureamide gemäß Formel I, Anspruch I, worin

$R^1$, $R^2$ gleich oder verschieden, aber immer ungleich Wasserstoff sind und unabhängig voneinander $C_{1-12}$-Alkyl gerade oder verzweigt, $C_{5-8}$-Cycloalkyl, $C_{5-8}$-Cycloalkylmethyl, Benzyl, Phenyl, durch Methyl, Methoxy oder Nitro substituiertes Phenyl oder zusammen eine $C_4$–$C_6$-Alkylenbrücke bedeuten und $R^3$, $R^4$ für Wasserstoff, Fluor, Chlor, Methyl, Methoxy, Trifluormethyl oder Nitro stehen.

3. Diselenobis-benzoesäureamide gemäß Formel I, Anspruch 1, worin
$R^1$ für Wasserstoff steht und
$R^2$ $C_{1-18}$-Alkyl gerade oder verzweigt, $C_{3-8}$-Cycloalkyl, $C_{3-8}$-Cycloalkylmethyl, Benzyl, Phenyl, ein- bis

dreimal unabhängig voneinander durch Fluor, Chlor, Brom, Methyl, Methoxy, Hydroxy, Trifluormethyl, Nitro, Dimethylamino, Cyan, Carboxy, Methoxycarbonyl, Ethoxycarbonylethyl bzw. Methylendioxy substituiertes Phenyl bedeutet und

$R^3$, $R^4$ für Wasserstoff, Fluor, Chlor, Methyl, Methoxy, Trifluormethyl, Nitro oder zusammen für Methylendioxy stehen.

4. Verfahren zur Herstellung von Verbindungen gemäß den Ansprüchen 1–3, dadurch gekennzeichnet, daß

a) Diselenobis-benzoesäuren der allgemeinen Formel II

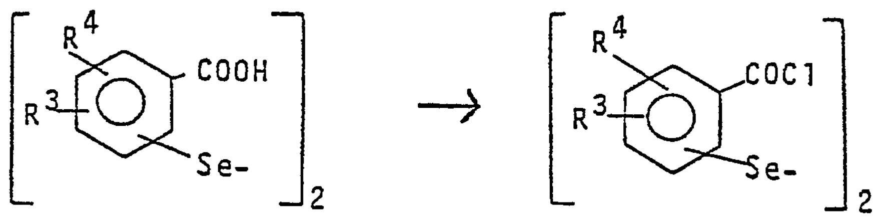

(II) (III)

worin $R^3$, $R^4$ die in Formel I angegebenen Bedeutungen haben und die Diselenobrücke sich in ortho-, meta- oder para-Stellung zur Carboxylgruppe befindet, in einem geeigneten Lösungsmittel mit Dichlormethylalkylether in Gegenwart von Zinkchlorid innerhalb von 72 Stunden bei Raumtemperatur in das entsprechende Säurechlorid der allgemeinen Formel III überführt wird, die nach Isolierung durch Umsetzung mit Aminen der allgemeinen Formel IV

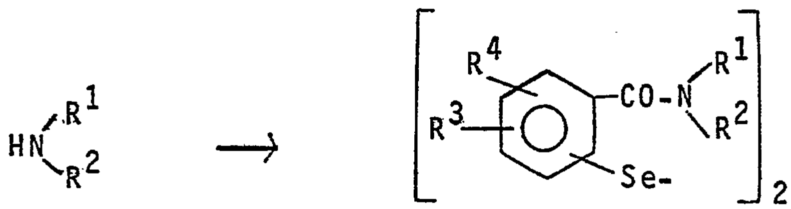

IV I

worin $R^1$ und $R^2$ die in Formel I angegebenen Bedeutungen haben, in die Verbindungen der Formel I übergeführt werden oder

b) Benzisoselenazolone der allgemeinen Formel V

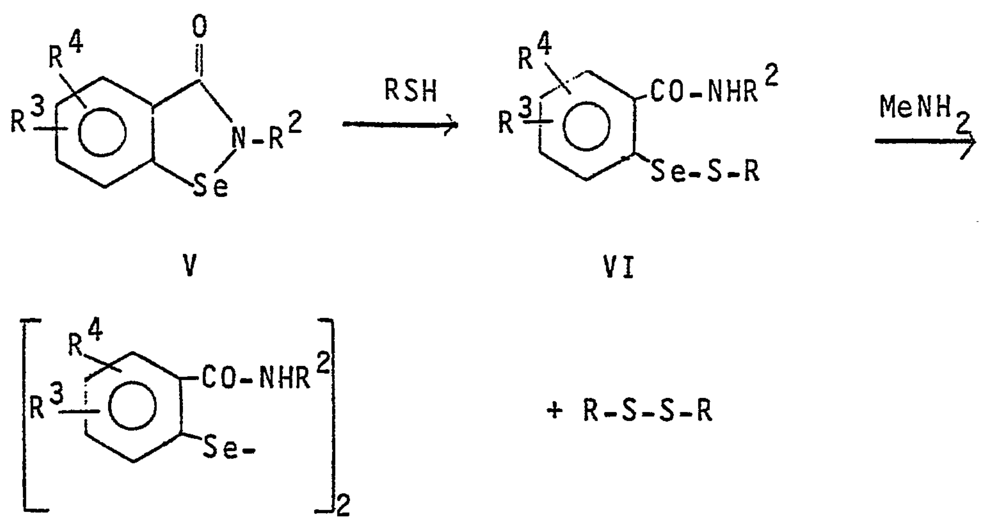


V VI

I

worin $R^2$, $R^3$, $R^4$ die in Formel I angegebenen Bedeutungen haben und R für Alkyl steht, in einem geeigneten organischen Lösungsmittel bei Raumtemperatur mit einer annähernd äquimolekularen Menge eines Alkylmercaptans zu den Zwischenverbindungen der Formel VI umsetzt, die bei Zugabe von Methylamin in die Diselenobis-benzoesäureamide der allgemeinen Formel I umgewandelt werden.

5. Pharmazeutische Präparate, dadurch gekennzeichnet, daß sie eine Verbindung der Formel I gemäß den Ansprüchen 1–3 als Wirkstoff im Gemisch mit üblichen pharmazeutischen Hilfs- und Trägerstoffen enthalten.

**Patentansprüche für den Vertragsstaat AT**

1. Verfahren zur Herstellung von Diselenobis-benzoesäureamiden der allgemeinen Formel I

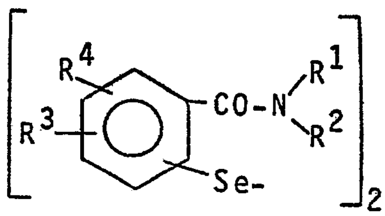

I

worin

$R^1$, $R^2$ gleich oder verschieden sind, mit der Maßgabe, daß mindestens ein Rest ungleich Wasserstoff sein muß, unabhängig voneinander Wasserstoff, $C_{1-18}$-Alkyl gerade oder verzweigt, $C_{3-8}$-Cycloalkyl, $C_{3-8}$-Cycloalkylmethyl, Phenylalkyl, Phenyl, ein- bis dreimal unabhängig voneinander durch Halogen, $C_{1-4}$-Alkyl,$C_{1-4}$-Alkoxy, Hydroxy, Trifluormethyl, Nitro, Di-($C_{1-4}$ alkyl)amino, Cyan, Carboxy, Alkoxycarbonyl, Alkoxycarbonylalkyl bzw. Methylendioxy substituiertes Phenyl bedeuten oder zusammen eine $C_{4-6}$-Alkylenbrücke bilden und

$R^3$, $R^4$ gleich oder verschieden sind und unabhängig voneinander für Wasserstoff, Halogen, $C_{1-4}$-Alkyl, $C_{1-4}$-Alkoxy, Trifluormethyl, Nitro oder zusammen für Methylendioxy stehen und die Diselenobrücke in beiden Ringen in ortho-, meta- oder para-Stellung zur Carbamidgruppe steht, dadurch gekennzeichnet, daß

a) Diselenobis-benzoesäuren der allgemeinen Formel II

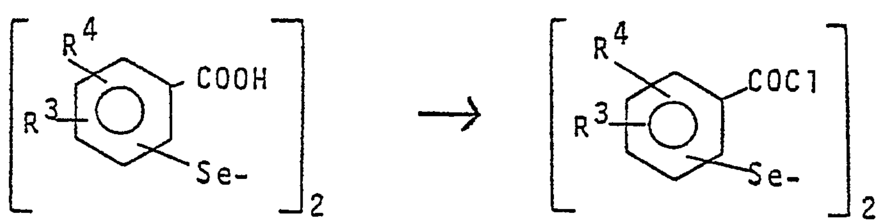

(II) (III)

worin $R^3$, $R^4$ die in Formel I angegebenen Bedeutungen haben und die Diselenobrücke sich in ortho-, meta- oder para-Stellung zur Carboxylgruppe befindet, in einem geeigneten Lösungsmittel mit Dichlormethylalkylether in Gegenwart von Zinkchlorid innerhalb von 72 Stunden bei Raumtemperatur in das entsprechende Säurechlorid der allgemeinen Formel III überführt wird, die nach Isolierung durch Umsetzung mit Aminen der allgemeinen Formel IV

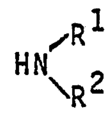

IV

worin $R^1$ und $R^2$ die in Formel I angegebenen Bedeutungen haben, in die Verbindungen der Formel I übergeführt werden oder

b) Benzisoselenazolone der allgemeinen Formel V

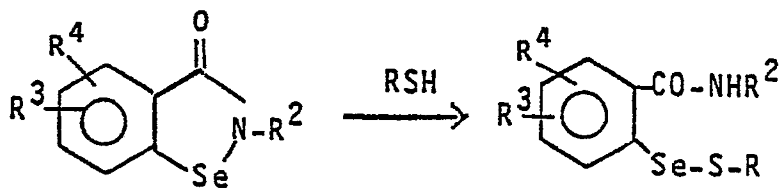


V VI

worin $R^2$, $R^3$, $R^4$ die in Formel I angegebenen Bedeutungen haben und R für Alkyl steht, in einem geeigneten organischen Lösungsmittel bei Raumtemperatur mit einer annähernd äquimolekularen Menge eines Alkylmercaptans zu den Zwischenverbindungen der Formel VI umsetzt, die bei Zugabe von Methylamin in die Diselenobisbenzoesäureamide der allgemeinen Formel I umgewandelt werden.

2. Verfahren gemäß Anspruch 1, dadurch gekennzeichnet, daß von Verbindungen der Formeln I, IV und V ausgegangen wird, worin

$R^1$, $R^2$ gleich oder verschieden, aber immer ungleich Wasserstoff sind und unabhängig voneinander $C_{1-12}$-Alkyl gerade oder verzweigt, $C_{5-8}$-Cycloalkyl, $C_{5-8}$-Cycloalkylmethyl, Benzyl, Phenyl, durch Methyl, Methoxy oder Nitro substituiertes Phenyl oder zusammen eine $C_4$–$C_6$-Alkylenbrücke bedeuten und

$R^3$, $R^4$ für Wasserstoff, Fluor, Chlor, Methyl, Methoxy, Trifluormethyl oder Nitro stehen.

3. Verfahren gemäß Anspruch 1, dadurch gekennzeichnet, daß von Verbindungen der Formeln I, IV und V ausgegangen wird, worin

$R^1$ für Wasserstoff steht und

$R^2$ $C_{1-18}$-Alkyl gerade oder verzweigt, $C_{3-8}$-Cycloalkyl, $C_{3-8}$-Cycloalkylmethyl, Benzyl, Phenyl, ein- bis dreimal unabhängig voneinander durch Fluor, Chlor, Brom, Methyl, Methoxy, Hydroxy, Trifluormethyl, Nitro, Dimethylamino, Cyan, Carboxy, Methoxycarbonyl, Ethoxycarbonylethyl bzw. Methylendioxy substituiertes Phenyl bedeutet und

$R^3$, $R^4$ für Wasserstoff, Fluor, Chlor, Methyl, Methoxy, Trifluormethyl, Nitro oder zusammen für Methylendioxy stehen.

**Claims for the Contracting States BE, CH, DE, FR, GB, IT, LI, LU, NL, SE**

1. Diselenobis-benzoic acid amides of the general formula (I):

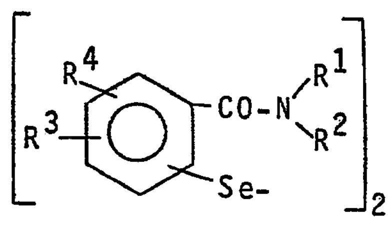

I

wherein:

$R^1$, $R^2$ are identical or different, with the provisio that at least one radical must not be hydrogen, and represent independently hydrogen, straight or branches $C_{1-18}$-alkyl, $C_{3-8}$-cycloalkyl, $C_{3-8}$-cycloalkylmethyl, phenylalkyl, phenyl, phenyl substituted once to three times independently by halogen, $C_{1-4}$-alkyl, $C_{1-4}$-alkoxy, hydroxy, trifluoromethyl, nitro, di( $C_{1-4}$-alkyl)-amino, cyano, carboxy, alkoxycarbonyl, alkoxycarbonylalkyl and methylenedioxy, respectively, or together form a $C_4$–$C_6$-alkylene bridge, and

$R^3$, $R^4$ are identical or different and represent independently hydrogen, halogen, $C_{1-4}$-alkyl, $C_{1-4}$-alkoxy, trifluoromethyl, nitro or together mean methylenedioxy,

the diseleno bridge being positioned in both rings in ortho-, meta- or para-position to the carbamido group.

2. Diselenobis-benzoic acid amides of formula (I) according to claim 1, wherein:

$R^1$, $R^2$ are identical or different but always do not mean hydrogen and represent independently straight or branched $C_{1-12}$-alkyl, $C_{5-8}$-cycloalkyl, $C_{5-8}$-cycloalkylmethyl, benzyl, phenyl, phenyl substituted by methyl, methoxy or nitro or together mean a $C_4$–$C_6$-alkylene bridge and

$R^3$, $R^4$ represent hydrogen, fluorine, chlorine, methyl, methoxy, trifluoromethyl or nitro.

3. Diselenobis-benzoic acid amides of formula (I) according to claim 1, wherein:

$R^1$ represents hydrogen and

$R^2$ represents straight or branched $C_{1-18}$-alkyl, $C_{3-8}$-cycloalkyl, $C_{3-8}$-cycloalkylmethyl, benzyl, phenyl,

phenyl substituted once to three times independently by fluorine, chlorine, bromine, methyl, methoxy, hydroxy, trifluoromethyl, nitro, dimethylamino, cyano, carboxy, methoxycarbonyl, ethoxycarbonylethyl and methylenedioxy, respectively, and
$R^3$, $R^4$ represent hydrogen, fluorine, chlorine, methyl, methoxy, trifluoromethyl, nitro or together represent methylenedioxy.

4. A process for the preparation of compounds according to claims 1 to 3, characterized in that
a) diselenobis-benzoic acids of the general formula (II):

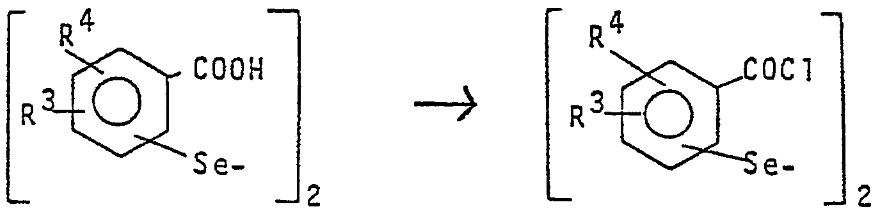

(II) (III)

wherein $R^3$, $R^4$ have the meanings given in formula (I) and wherein the diseleno bridge is in ortho-, meta- or para-position to the carboxyl group, in a suitable solvent by means of dichloromethylalkyl ethers in the presence of zinc chloride within 72 hours at room temperature are converted into the corresponding acid chlorides of the general formula (III) which, after isolating them, by reacting them with amines of the general formula (IV)

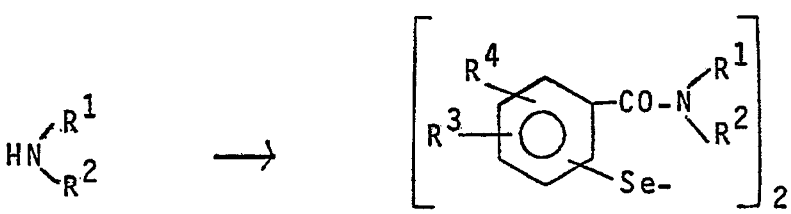

IV I

wherein $R^1$ and $R^2$ have the meanings given in formula (I) are converted into the compounds of formula (I) or
b) benzoisoselenazolones of the general formula (V):

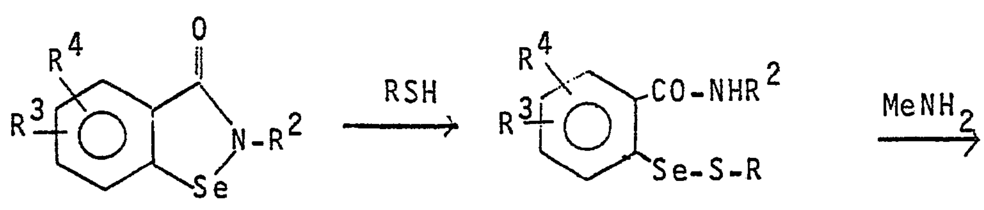


V VI

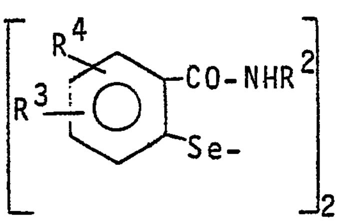

+ R-S-S-R

I

wherein $R^2$, $R^3$, $R^4$ have the meanings given in formula (I) and wherein R represents alkyl, in a suitable

organic solvent at room temperature are reacted with an approximately equimolecular amount of an alkyl mercaptane forming the intermediate compounds of the formula (VI) which by the addition of methylamine are converted into the diselenobis-benzoic acid amides of the general formula (I).

5. Pharmaceutical preparations, characterized in that they contain a compound of formula (I) according to the claims 1 to 3 as active component in admixture with common pharmaceutical excipients and carriers.

**Claims for the Contracting State AT**

1. A process for the preparation of diselenobis-benzoic acid amides of the general formula (I)

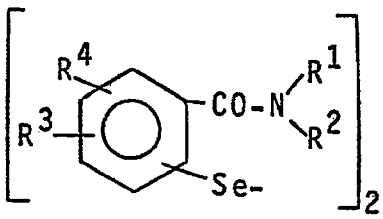

I

wherein
$R^1$, $R^2$ are identical or different, with the provisio that at least one radical must not be hydrogen, and represent independently hydrogen, straight or branched $C_{1-18}$-alkyl, $C_{3-8}$-cycloalkyl, $C_{3-8}$-cycloalkylmethyl, phenylalkyl, phenyl, phenyl substituted once to three times independently by halogen, $C_{1-4}$-alkyl, $C_{1-4}$-alkoxy, hydroxy, trifluormethyl, nitro, di-($C_{1-4}$-alkyl)-amino, cyano, carboxy, alkoxycarbonyl, alkoxycarbonylalkyl and methylenedioxy, respectively, or together from a $C_{4-6}$-alkylene bridge, and
$R^3$, $R^4$ are identical or different and represent independently hydrogen, halogen, $C_{1-4}$-alkyl, $C_{1-4}$-alkoxy, trifluormethyl, nitro or together mean methylenedioxy,
the diseleno bridge being positioned in both rings in ortho-, meta- or para-position to the carbamido group, characterized in that

a) diselenobis-benzoic acids of the general formula (II)

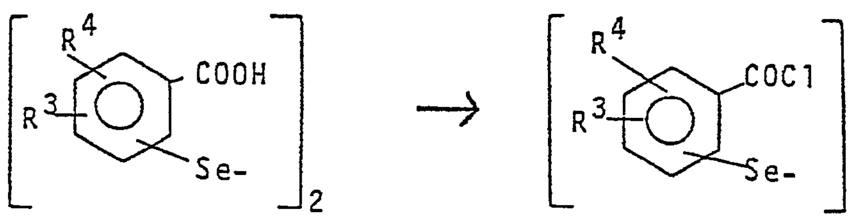

(II) (III)

wherein $R^3$, $R^4$ have the meanings given in formula (I) and wherein the diselen bridge is in ortho-, meta- or para-position to the carboxyl group, in a suitable solvent by means of dichloromethylalkyl ethers in the presence of zinc chloride within 72 hours at room temperature are converted into the corresponding acid chlorides of the general formula (III) which, after isolating them, by reacting them with amines of the general formula (IV)

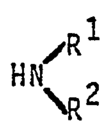

(IV)

wherein $R^1$ und $R^2$ have the meanings given in formula (I) are converted into the compounds of formula (I) or

b) benzisoselenazolones of the general formula (V)

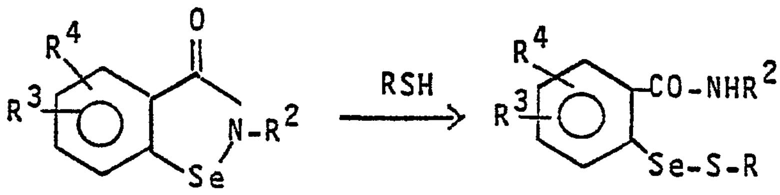

V VI

wherein $R^2$, $R^3$, $R^4$ have the meanings given in formula (I) and wherein R represents alkyl, in a suitable organic solvent at room temperature are reacted with an approximately equimolecular amount of an alkyl mercaptane forming the intermediate compounds of the formula (VI) which by the addition of methylamine are converted into the diselenobis-benzoic acid amides of the general formula (I).

2. A process as claimed in claim 1, characterized in that it is started from compounds of formulas I, IV und V wherein

$R^1$, $R^2$ are identical or different but always do not mean hydrogen and represent independently straight or branched $C_{1-12}$-alkyl, $C_{5-8}$-cycloalkyl, $C_{5-8}$-cycloalkylmethyl, benzyl, phenyl, phenyl substituted by methyl, methoxy or nitro or together mean a $C_4$–$C_6$-alkylene bridge and

$R^3$, $R^4$ represent hydrogen, fluorine, chlorine, methyl, methoxy, trifluoromethyl or nitro.

3. A process as claimed in claim 1, characterized in that it is started from compounds of formulas I, IV und V wherein

$R^1$, represents hydrogen and

$R^2$ represents straight or branched $C_{1-18}$-alkyl, $C_{3-8}$-cycloalkyl, $C_{3-8}$-cycloalkylmethyl, benzyl, phenyl, phenyl substituted once to three times independently by fluorine, chlorine, bromine, methyl, methoxy, hydroxy, trifluoromethyl, nitro, dimethylamino, cyano, carboxy, methoxycarbonyl, ethoxycarbonylethyl and methylenedioxy, respectively, and

$R^3$, $R^4$ represent hydrogen, fluorine, chlorine, methyl, methoxy, trifluoromethyl, nitro or together represent methylenedioxy.

**Revendications pour les Etats contractants BE, CH, DE, FR, GB, IT, LI, LU, NL, SE**

1. Disélénobis-benzoamide de formule générale I

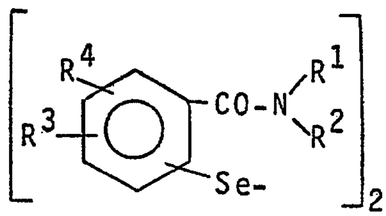

I

dans laquelle

$R^1$, $R^2$ sont identiques ou différents avec la condition qu'au moins un reste est différent de l'hydrogène et représentent indépendamment l'un de l'autre l'hydrogène, un alkyle $C_{1-18}$ linéaire ou ramifié, un cycloalkyle $C_{3-8}$, cycloalkylméthyle $C_{3-8}$, phénylalkyle, phényle, phényle substitué de une à trois fois indépendamment l'un de l'autre par un halogène, alkyle $C_{1-4}$, alcoxy $C_{1-4}$, hydroxy, trifluorométhyle, nitro, di-(alkyle $C_{1-4}$)-amino, cyano, carboxy, alcoxycarbonyle, alcoxycarbonylalkyle ou méthylènedioxy ou forment ensemble une chaîne alkylène $C_4$–$C_6$, et

$R^3$, $R^4$ sont identiques ou différents et représentent indépendamment l'un de l'autre un hydrogène, halogène, alkyle $C_{1-4}$, alcoxy $C_{1-4}$, trifluorométhyle, nitro ou forment ensemble le méthylènedioxy, et le pont diséléno se trouve en position ortho, méta ou para vis-à-vis du groupe carbamide.

2. Disélénobis-benzamides de formule I, selon la revendication 1, dans laquelle

$R^1$, $R^2$ sont identiques ou différents, mais toujours différents de l'hydrogène et représentent indépendamment l'un de l'autre, un alkyle $C_{1-12}$ linéaire ou ramifié, un cycloalkyle $C_{5-8}$, cycloalkylméthyle $C_{5-8}$, benzyle, phényle, phényle substitué par un méthyle, méthoxy ou nitro ou représentent ensemble un pont alkylène $C_4$–$C_6$ et

$R^3$, $R^4$ sont l'hydrogène, le fluor, chlore, méthyle, méthoxy, trifluorométhyle ou nitro.

3. Disélénobisbenzamides de formule I, selon la revendication 1, dans laquelle

$R^1$ est l'hydrogène et

$R^2$ est un alkyle $C_{1-18}$ linéaire ou ramifié, cycloalkyle $C_{3-8}$, cycloalkylméthyle $C_{3-8}$, benzyle, phényle,

phényle substitué de une à trois fois indépendamment l'un de l'autre par le fluor, chlore, brome, méthyle, méthoxy, hydroxy, trifluorométhyle, nitro, diméthylamino, cyano, carboxy, méthoxycarbonyle, éthoxycarbonyléthyle ou méthylènedioxy, et

$R^3$, $R^4$ sont l'hydrogène, le fluor, chlore, méthyle, méthoxy, trifluorométhyle, nitro ou forment ensemble le méthylènedioxy.

4. Procédé de préparation de composés selon les revendications 1–3, caractérisé en ce qu'on transforme

a) l'acide disélénobisbenzoïque de formule générale II

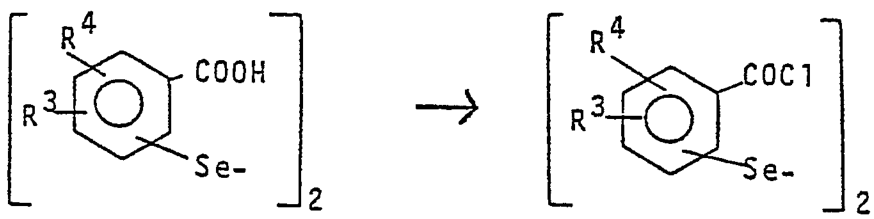

(II) (III)

dans laquelle $R^3$, $R^4$ ont les significations données dans la Formule I, et le pont diséléno se trouve en position ortho, méta ou para, dans un solvant approprié, avec de l'éther dichlorométhylalkylique, en présence de chlorure de zinc en l'espace de 72 heures, à température ambiante, pour donner les chlorures d'acides correspondants de formule générale III, qui après isolement sont transformés en les composés de formule I, selon les procédés connus en soi, par réaction avec les amines de formule générale IV

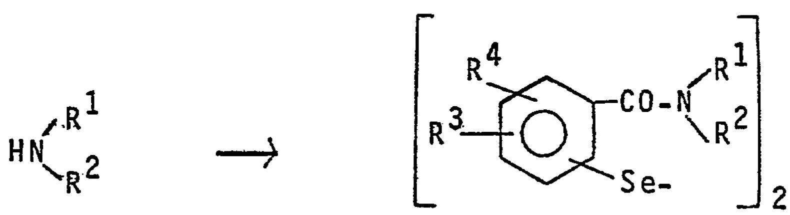

IV I

dans lesquelles $R^1$, $R^2$ ont les significations données pour les composés de formule I, ou

b) on fait réagir des benzoisosélénazolones de formule générale V

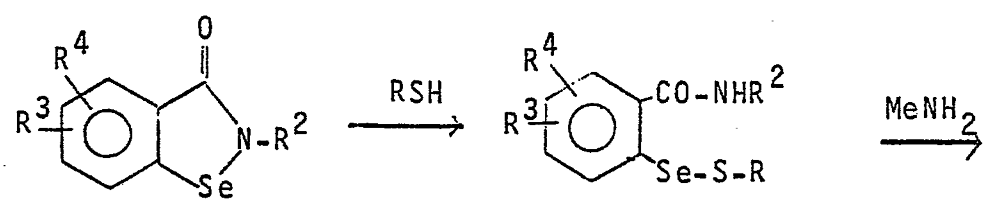


V VI

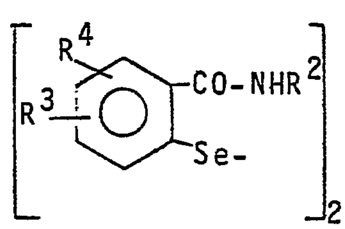

+ R-S-S-R

I

dans laquelle $R^2$, $R^3$, $R^4$ ont les significations données pour la formule I et R représente un alkyle, dans un solvant organique approprié, à température ambiante, avec une quantité presque équimoléculaire d'un alkylmercaptan pour donner les composés intermédiaires de formule VI, qui sont transformés, par addition de méthylamine en les disélénobisbenzamides de formule générale I.

5. Préparations pharmaceutiques, caractérisées en ce qu'elles contiennent un composé de formule I selon les revendications 1–3 comme principe actif en mélange avec les produits auxiliaires et véhicules pharmaceutiques usuels.

**Revendications pour l'Etat contractant AT**

1. Procédé de préparation de disélénobis-benzamides de formule générale I

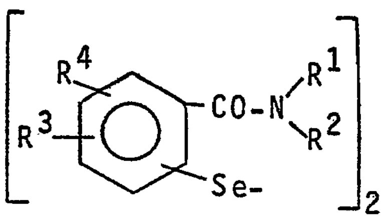

I

dans laquelle
$R^1$, $R^2$ sont identiques ou différents avec la condition qu'au moins un reste est différent de l'hydrogène et représentent indépendamment l'un de l'autre, l'hydrogène, un alkyle $C_{1-18}$ linéaire ou ramifié, un cycloalkyle $C_{3-8}$, cycloalkylméthyle $C_{3-8}$, phénylalkyle, phényle, phényle substitué de une à trois fois indépendamment l'un de l'autre par un halogène, alkyle $C_{1-4}$, alcoxy $C_{1-4}$, hydroxy, trifluorométhyle, nitro, di-(alkyle $C_{1-4}$)-amino, cyano, carboxy, alcoxycarbonyle, alcoxycarbonylalkyle ou méthylènedioxy ou forment ensemble une chaîne alkylène $C_4$–$C_6$, et
$R^3$, $R^4$ sont identiques ou différents et représentent indépendamment l'un de l'autre un hydrogène, halogène, alkyle $C_{1-4}$, alcoxy $C_{1-4}$, trifluorométhyle, nitro ou forment ensemble le méthylènedioxy, et le pont diséléno se trouve en position ortho, méta ou para vis-à-vis du groupe carbamide, caractérisé en ce qu'on transforme

a) l'acide disélénobisbenzoïque de formule générale II

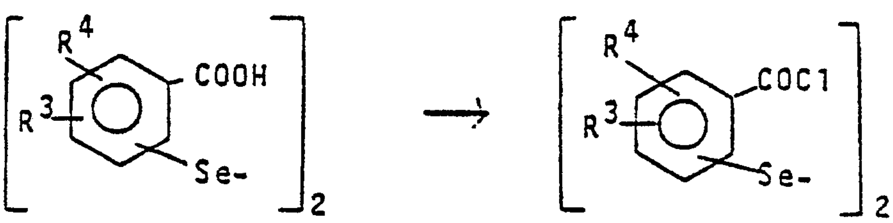

II III

dans laquelle $R^3$, $R^4$ ont les significations données dans la Formule I, et le pont diséléno se trouve en position ortho, méta ou para, dans un solvant approprié, avec de l'éther dichlorométhylalkylique, en présence de chlorure de zinc, en l'espace de 72 heures, à température ambiante, pour donner les chlorures d'acides correspondants de formule générale III, qui après isolement sont transformés en les composés de formule I selon les procédés connus en soi, par réaction avec les amines de formule générale IV

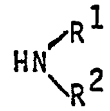

IV

dans laquelle $R^1$, $R^2$ ont les significations données pour les composés de formule I, ou

b) on fait réagir des benzoisosélénazolones de formule générale V

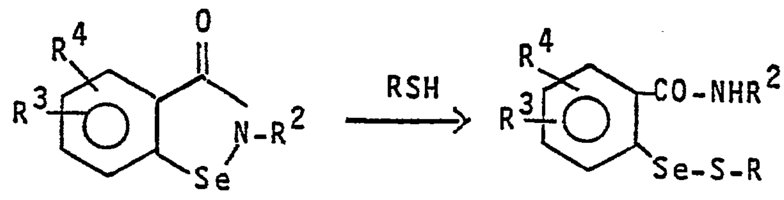

V

VI

dans laquelle $R^2$, $R^3$, $R^4$ ont les significations données pour la formule I et R représente un alkyle, dans un solvant organique approprié, à température ambiante, avec une quantité presqué équimoléculaire d'un alkylmercaptan pour donner les composés intermédiaires de formula VI, qui sont transformés, par addition de méthylamine en les disélénobis-benzamides de formule générale I.

2. Procédé selon la revendication 1, caractérisé en ce qu'on part de composés de formules I, IV et V dans lesquelles

$R^1$, $R^2$ sont identiques ou différents, mais toujours différents de l'hydrogène et représentent indépendamment l'un de l'autre un alkyle $C_{1-12}$ linéaire ou ramifiée, un cycloalkyle $C_{5-8}$, cycloalkylméthyle $C_5$68, benzyle, phényle, phényle substitué par un méthyle, méthoxy ou nitro ou représentent ensemble un pont alkylène $C_4$–$C_6$ et

$R^3$, $R^4$ sont l'hydrogène, le fluor, chlore, méthyle, méthoxy, trifluorométhyle ou nitro.

3. Procédé selon la revendication 1, caractérisé en ce qu'on part de composés de formules I, IV et V, dans laquelle

$R^1$ est l'hydrogène et

$R^2$ est un alkyle $C_{1-18}$, linéaire ou ramifié, cycloalkyle $C_{3-8}$ , cycloalkylméthyle $C_{3-8}$, benzyle, phényle, phényle substitué de une à trois fois indépendamment l'un de l'autre par le fluor, chlore, brome, méthyle, méthoxy, hydroxy, trifluorométhyle, nitro, diméthylamino, cyano, carboxy, méthoxycarbonyle, éthoxycarbonyléthyle ou méthylènedioxy, et

$R^3$, $R^4$ sont l'hydrogène, le fluor, chlore, méthyle, méthoxy, trifluorométhyle, nitro ou forment ensemble le méthylènedioxy.